(19) Europäisches Patentamt
European Patent Office
Office européen des brevets

(11) **EP 2 011 816 B1**

(12) **FASCICULE DE BREVET EUROPEEN**

(45) Date de publication et mention
de la délivrance du brevet:
**21.09.2016 Bulletin 2016/38**

(21) Numéro de dépôt: **08356103.5**

(22) Date de dépôt: **04.07.2008**

(51) Int Cl.:
*A61L 27/20* (2006.01)  *A61L 27/52* (2006.01)
*A61L 27/50* (2006.01)  *A61L 27/48* (2006.01)
*C08B 37/08* (2006.01)  *C08J 3/075* (2006.01)
*C08J 3/24* (2006.01)  *C08L 5/08* (2006.01)

(54) **Gel co-réticulé de polysaccharides**

Vernetztes Polysaccharidgel

Co-reticulated polysaccharide gel

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **05.07.2007 FR 0704860**

(43) Date de publication de la demande:
**07.01.2009 Bulletin 2009/02**

(73) Titulaire: **Laboratoires Vivacy
74160 Archamps (FR)**

(72) Inventeur: **Estelle Piron
73490 La Ravoire (FR)**

(74) Mandataire: **Tripoz, Inès
Cabinet Tripoz
Le Pôle Sud
22 rue Seguin
69002 Lyon (FR)**

(56) Documents cités:
**WO-A-97/04012        WO-A-2008/059058
FR-A- 2 733 427        FR-A- 2 780 730
FR-A- 2 865 737        US-A1- 2004 127 698
US-A1- 2005 069 572    US-B1- 6 638 538**

Remarques:
Le dossier contient des informations techniques
présentées postérieurement au dépôt de la demande
et ne figurant pas dans le présent fascicule.

EP 2 011 816 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne des gels injectables de polysaccharides co-réticulés, le procédé de préparation desdits gels injectables, ainsi que leurs utilisations notamment en chirurgie réparatrice, en chirurgie esthétique, et en chirurgie de comblement des tissus biologiques.

Les gels (et hydrogels, lorsque les gels sont présents en milieu aqueux) injectables de polysaccharides résorbables (ou biodégradables) ou de polymères permanents sont connus et utilisés dans de larges domaines thérapeutiques et esthétiques depuis de longues années.

Ainsi des gels de polymères biodégradables sont injectés dans l'articulation du genou ou de la hanche en cas d'arthrose pour supplémenter le liquide synovial déficient et restaurer temporairement les propriétés chondroprotectrices dudit liquide biologique.

Ces mêmes solutions viscoélastiques injectables peuvent été utilisées pour optimiser la chirurgie de la presbytie, suite aux incisions sclérales permettant de modifier la courbure de l'oeil. En effet, l'injection de tels gels biodégradables au coeur desdites incisions permettra de supprimer la cicatrisation « bord à bord » des incisions ou « rétraction » qui annulerait le bénéfice de la chirurgie.

Si la durée de rémanence du gel biodégradable est suffisante, la cicatrisation aura eu lieu avant la disparition finale du gel. Ce dernier permettra donc de garantir une bonne cicatrisation des incisions sans rétraction ainsi qu'une cicatrisation optimale de la cornée. Le gel viscoélastique utilisé devra cependant être cohésif et suffisamment rémanent pour garantir la réussite de l'opération.

Dans le même type d'application, des solutions viscoélastiques sont injectées pour séparer certains tissus. Ces solutions injectables sont par exemple utilisées pour éviter des adhésions péritonéales suite à une intervention chirurgicale, mais aussi dans le sphincter, l'urètre.

Enfin, ce type de solutions viscoélastiques est particulièrement utilisé en chirurgie esthétique ou réparatrice, afin de combler les rides ou les défauts cutanés, pour augmenter le volume des lèvres, des pommettes ou du menton, et pour la correction des cicatrices.

Dans ce domaine de comblement des rides et défauts cutanés, deux types principaux de produits sont utilisés, ceux à base de produits ou polymères permanents, type gel de silicone ou gel de polyacrylamide, et ceux à base de produits biorésorbables comme le collagène ou l'acide hyaluronique, ainsi que ses sels et dérivés, réticulés ou non.

Les produits biphasiques sont une variante des solutions ci-dessus et consistent à injecter des particules permanentes ou semi-permanentes préalablement dispersées dans un gel biorésorbable qui sert de matrice d'injection.

Les produits permanents sont sujets à controverse suite aux réactions secondaires qu'ils peuvent générer, de type granulomes, nodules ou ulcères de la peau, liés la plupart du temps au phénomène de rejet à corps étrangers. Ces réactions secondaires peuvent apparaître très tardivement et sont alors particulièrement difficiles à traiter. La migration éventuelle des produits dans des tissus sous-jacents peut également être très néfaste (voir « Management of complications after implantation of fillers », K. De Boulle, Journal of Cosmet. Derm., 3, (2004), 2-15 ; « Foreign body granulomas due to injectable aesthetic microimplants », C. Rudolph et P. Soyer, Surg. Pathol., 23, (1999), 113-7).

Ainsi les injections de silicone, très utilisées auparavant, ont parfois été la cause de siliconomes (nodules inflammatoires) (voir « Adverse reactions to injectable soft tissue permanent fillers », L. Christensen et V. Breiting, Aesthetic Plastic Surgery, 29, (2005), 34-48), voire de réactions allergiques tardives. De même des réactions de nature infectieuse ont été observées dans les premières années qui ont suivi l'injection de gel polyacrylamide (APS, 29, (2005), 34-48).

Il en est de même pour les injections de produits contenant des particules solides, celles-ci, si elles sont non résorbables ou à cinétique de résorption très lente (tel le PLA, environ 5 ans) peuvent dans certains cas entraîner des réactions inflammatoires chroniques, voire l'apparition de granulomes.

Le principe de ces gels avec particules est basé sur une réponse à corps étrangers. En effet, ces particules sont censées générer une réaction inflammatoire à corps étranger supposée stimuler la synthèse de nouvelles fibres de collagène en induisant une prolifération des fibroblastes, voire une fibrose, ce qui participera à la création de volumes (« Management of complications after implantation of fillers », K. De Boulle, Journal of Cosmet. Derm. , 3, (2004), 2-15).

Cependant la difficulté est de trouver le bon équilibre entre la génération d'une prolifération légère et maîtrisée de fibroblastes, et l'apparition de réactions inflammatoires trop fortes générant des infections, des granulômes ou une fibrose trop importante, non maîtrisée, qui deviendra gênante.

Les réactions inflammatoires sont également directement liées à la taille des particules et à leur migration potentielle hors du site d'injection. Ainsi les particules inférieures à 10 $\mu$m sont inadaptées à l'injection sous-cutanée car elles vont pouvoir migrer plus facilement en dehors du site ; elles seront également phagocytées très rapidement.

Cependant, dans le cas d'injections à visée esthétique (comblement des rides et défauts cutanés, augmentation du volume des lèvres), la taille des particules ne doit pas non plus être trop importante afin de pouvoir être injectées à travers des aiguilles de diamètre fin. Le compromis est donc difficile à maîtriser industriellement.

Les produits injectables avec particules, tels qu'ils viennent d'être définis, se positionnent sur deux marchés complémentaires de l'esthétique :

- les produits de comblement des rides profondes, et
- les « volumateurs », qui sont destinés à redonner du volume dans les pommettes, le menton, etc...

Cependant du fait des effets secondaires liés aux particules, à leur migration potentielle, et/ou à l'aspect permanent de certains implants, les praticiens injecteurs préconisent de plus en plus des implants résorbables exempt de particules.

Parmi les produits résorbables, le collagène a été largement utilisé. Il a toutefois été montré qu'il pouvait être parfois à l'origine de réactions allergiques. Pour cette raison, il a été progressivement délaissé au profit des sels d'acide hyaluronique. Cette mise à l'écart trouve également une autre raison dans son origine bovine, avec les risques connus aujourd'hui qu'elle peut entraîner.

Les gels d'acide hyaluronique, en particulier ses sels et/ou dérivés, réticulés ont donc pris une part importante sur le marché du comblement des rides et défauts cutanés. Ils bénéficient d'une rémanence accrue suite à la réticulation du polysaccharide, et sont bien perçus du fait de leur bonne tolérance liée à l'innocuité et à la pureté du polysaccharide, d'origine non animale pour la majorité des produits.

Cependant, afin d'augmenter la rémanence du gel injecté dans les tissus, de forts taux de réticulation sont souvent utilisés, rendant le gel difficile à injecter et limitant la précision du geste chirurgical, pouvant même générer plus d'ecchymoses, ou d'oedèmes post-injection qu'habituellement, ce qui ne satisfait ni les médecins ni les sujets traités.

La demande de brevet CA 2 561 685 décrit des particules de matériau viscoélastique, sous forme de particules de gel, injectables, lorsqu'elles sont soumises à une solution saline physiologique.

Dans la demande de brevet EP 0 466 300 est décrit un gel biphasique, la première phase comprenant des particules d'un gel polymère gonflé uniformément distribuées, par simple mélange, dans une solution viscoélastique d'un polymère formant la seconde phase.

La demande de brevet FR 2 865 737 divulgue un procédé de fabrication d'un gel réticulé biocompatible, ledit procédé comprenant la réticulation d'une quantité prédéterminée d'au moins un polymère biocompatible, puis l'ajout d'une quantité supplémentaire de polymère, avec dilution et réticulation. Le gel réticulé biocompatible obtenu est un gel dont le degré de réticulation varie, avec des zones fortement réticulées dont le taux de réticulation diminue progressivement. Aucune maîtrise de la répartition des zones plus fortement réticulées n'est cependant assurée par ce biais. Ainsi le gel final obtenu présentera des zones très fortement réticulées et d'autres plus faiblement qui vont générer des forces d'injection parfois très élevées, parfois plus faibles.

Un premier objectif de la présente invention consiste à proposer un gel injectable permettant de s'affranchir des défauts et inconvénients des gels injectables connus aujourd'hui et utilisés notamment dans les domaines de la chirurgie réparatrice, chirurgie esthétique, chirurgie de comblement des tissus biologiques et autres.

Plus particulièrement, la présente invention a pour objectif des gels injectables comportant des particules « liées » au gel, permettant de garantir une rémanence accrue et suffisante pour la pathologie ou le défaut traité, rémanence alliée à une facilité d'injection au cours de l'acte clinique, même à travers de fines aiguilles comme généralement requis en chirurgie esthétique.

Un autre objectif de l'invention consiste à proposer un gel cohésif, afin d'assurer son maintien sur le site d'injection et d'éviter sa migration dans les tissus voisins, tout en garantissant un bon remplissage du volume visé.

Selon un autre objectif, l'invention propose un produit sous forme de gel présentant une double action :

- un comblement immédiat et durable ; et
- un phénomène de relance fibroblastique suite à une réaction légère à corps étrangers (les particules ou fragments de gel) permettra d'augmenter le volume sous-cutané.

D'autres objectifs encore apparaîtront à la lumière de la description de l'invention qui suit.

La Demanderesse a maintenant découvert que ces objectifs sont atteints en totalité ou en partie grâce aux gels cohésifs injectables de polysaccharides co-réticulés tel que défini dans les revendications 1-3.

Par « fortement » et « faiblement », on entend que le taux de réticulation du premier gel de polysaccharide fortement réticulé est supérieur à celui du second gel de polysaccharide faiblement réticulé.

Le polysaccharide constituant le gel fortement réticulé peut être identique à, ou différent du polysaccharide constituant le gel faiblement réticulé.

Les polysaccharides mis en oeuvre dans le gel de la présente invention sont l'acide hyaluronique et ses sels, en particulier ses sels acceptables du point de vue physiologique, tels que les sels de sodium, potassium, calcium, avantageusement le sel de sodium. Sont également avantageusement utilisés la chondroïtine sulfate et ses sels et les dérivés cellulosiques tel que l'hydroxypropylméthylcellulose, et les mélanges de deux ou plusieurs d'entre eux. De manière particulièrement préférée, le gel fortement réticulé et le gel faiblement réticulé sont à base de hyaluronate de sodium.

En effet, le hyaluronate de sodium présente des propriétés particulièrement avantageuses en raison de son recul important d'utilisation en injection intradermique, intra-articulaire, intrapéritonéale, et autres, ainsi que pour ses excellentes propriétés rhéologiques.

Le gel selon la présente invention comprend donc un premier polysaccharide fortement réticulé et un second polysaccharide faiblement réticulé, lesdits premier et second polysaccharide étant liés entre eux par liaisons de covalence, c'est-à-dire que les deux polysaccharides sont co-réticulés.

Le polysaccharide fortement réticulé se présente sous la forme de particules, de taille généralement comprise entre 10 $\mu$m et 5 mm, de préférence entre 20 $\mu$m et 1 mm, idéalement entre 50 $\mu$m et 500 $\mu$m, et réparties de manière homogène dans le polysaccharide faiblement réticulé (la matrice), lesdites particules de polysaccharide fortement réticulé étant liées de manière covalente (ou encore co-réticulées) à la matrice (polysaccharide faiblement réticulé). Ainsi les particules ou fragments de gel ne pourront en aucun cas migrer hors du site d'injection. Par « particule », on désigne un fragment de polymère qui est par exemple sous forme sphérique ou de bâtonnets. De manière générale, la forme de ces particules n'est pas régulière. Les particules peuvent notamment être poreuses. Les tailles indiquées ci-dessus sont des dimensions moyennes, et peuvent correspondre aux particules hydratées ou sèches. Ces particules sont obtenues par broyage du polysaccharide fortement réticulé hydraté ou sec. La forme et la taille des particules dépendent donc de la durée et de la technique du broyage.

L'agent de co-réticulation permettant la formation des liaisons de covalence entre les particules de polymère fortement réticulé et la matrice de polysaccharide faiblement réticulé, est de tout type connu en soi et peut par exemple être avantageusement choisi parmi les agents de réticulations eux-mêmes connus pour effectuer les réticulation des polysaccharides, comme on le verra plus loin.

Il doit être compris que le gel selon la présente invention comprend deux gels distincts, l'un fortement réticulé, l'autre faiblement réticulé, les deux gels étant liés entre eux par liaison de covalence, le gel faiblement réticulé présentant au moins une zone dans laquelle le taux de réticulation (noté X2 dans la suite) est substantiellement constant. Avantageusement, le gel faiblement réticulé présente au moins une zone dans laquelle le taux de réticulation (X2) est substantiellement constant et le gel fortement réticulé présente au moins une zone dans laquelle le taux de réticulation (noté X1 dans la suite) est substantiellement constant, avec X1 supérieur à X2.

Par l'intermédiaire de particules de gel fortement réticulé, elles-mêmes co-réticulées au sein d'une matrice plus faiblement réticulé, le gel selon la présente invention présente une double action : comblement immédiat et durable dû au gel final (matrice et particules co-réticulées au sein de cette matrice) et dans un deuxième temps, lorsque la matrice plus faiblement réticulée est biodégradée dans les tissus, phénomène de relance fibroblastique suite à une réaction légère à corps étrangers (les particules) entraînant une augmentation du volume sous-cutané.

En effet, les particules des polysaccharide fortement réticulées ont un aspect souple et conformable, celles-ci étant des particules de gel totalement biocompatibles (résorbables) et non des particules rigides, susceptibles d'entraîner une très légère réaction à corps étranger et ainsi d'éviter les complications décrites plus haut avec les produits biphasiques à particules solides et plus ou moins rigides. De plus, la co-réticulation entre les particules de gel fortement réticulé et le second gel réticulé permet d'éviter les risques de migration de particules hors du site d'injection.

Selon la présente invention, le gel de polysaccharides co-réticulés conduit, après hydratation dans un tampon isotonique, à un hydrogel cohésif composé d'une matrice d'un ou plusieurs polysaccharides, tels que définis précédemment, ladite matrice étant réticulée avec des particules de ce même polysaccharide, ou d'un ou plusieurs autres polysaccharides, fortement réticulées et liées à cette matrice. Cet hydrogel est injectable même à travers des aiguilles de fin diamètre.

Le taux de réticulation d'une part du polysaccharide fortement réticulé et d'autre part du polysaccharide faiblement réticulé sont des paramètres importants à respecter : le taux de réticulation du polysaccharide fortement réticulé doit être suffisamment élevé pour garantir un gel selon l'invention qui soit cohésif, et le plus rémanent possible. D'autre part, le taux de réticulation du polysaccharide faiblement réticulé doit être suffisamment élevé pour garantir un gel cohésif, monophasique, et qui reste implanté au site d'injection sans se propager hors dudit site, sans toutefois être trop élevé pour une utilisation aisée notamment par injection.

Dans la présente invention, on définit un premier taux de réticulation X1 initial, désigné comme X1 dans la suite de la demande, caractéristique du gel fortement réticulé obtenu par réticulation du premier polysaccharide, et qui est ensuite réduit en particules de gel réticulé (comme on le verra plus loin), et un deuxième taux de réticulation X2, caractéristique de la matrice de polysaccharides incorporant les particules qui seront réticulées en son sein. Un taux de réticulation X1' est défini comme étant le taux de réticulation du premier polysaccharide après réticulation en présence du premier polysaccharide et du second polysaccharide.

Chacun des taux de réticulation X1 et X2 est défini comme étant égal au rapport :

$$X = \frac{\text{nombre de moles de réticulant introduites dans le milieu réactionnel}}{\text{nombre total de motifs disaccharides introduits dans le milieu réactionnel}}$$

Typiquement, le polysaccharide fortement réticulé présente un taux de réticulation X1 compris entre environ 0,2 et environ 0,7, préférentiellement entre environ 0,22 et environ 0,6, notamment 0,25 à 0,45. Quant au polysaccharide faiblement réticulé, son taux de réticulation X2 est typiquement compris entre environ 0,03 et environ 0,3, de préférence entre environ 0,05 et environ 0,25, notamment 0,06 à 0,18. X1' est supérieur ou égal à X1 et est compris entre 0,21 et 0,9.

Dans le gel de la présente invention, le rapport pondéral entre le polysaccharide fortement réticulé et le polysaccharide faiblement réticulé peut varier dans de très larges proportions, selon la nature des polysaccharides utilisés, leurs taux de réticulation respectifs, et également selon l'utilisation envisagée.

Généralement la proportion pondérale des particules de polysaccharide fortement réticulé dans le produit fini est comprise entre environ 0,5 à 99 %, préférentiellement de 5 à 60 %, plus préférentiellement de 10 à 40 %. Par « produit fini », on entend le produit prêt à être conditionné dans une seringue.

L'invention concerne également les compositions comprenant un gel tel que défini ci-dessus.

Selon un second aspect, la présente invention concerne le procédé de préparation du gel injectable co-réticulé, tel qu'il vient d'être décrit, ledit procédé comprenant les étapes telles que définies à la revendication 5.

Les particules de premier polysaccharide fortement réticulé de l'étape 1) ont un taux de réticulation X1. Ce taux de réticulation est susceptible d'être modifié lors de l'étape 4). A l'issue de l'étape 4), le taux de réticulation du premier polysaccharide est X1', avec X1' supérieur ou égal à X1.

Les particules de premier polysaccharide fortement réticulé fournies lors de l'étape 1) peuvent être sèches ou hydratées.

Les particules de premier polysaccharide fortement réticulé sont susceptibles d'être obtenues par un procédé comprenant les étapes suivantes :

1') réticulation en milieu aqueux en présence d'un agent réticulant d'au moins un premier polysaccharide pour obtenir un premier polysaccharide réticulé hydraté ayant un taux de réticulation X1 ;

2') éventuellement séchage du premier polysaccharide réticulé hydraté obtenu lors de l'étape 1') pour obtenir un premier polysaccharide réticulé sec,

3') broyage dudit premier polysaccharide réticulé obtenu lors de l'étape 1') ou 2') en particules de granulométrie souhaitée pour obtenir des particules de premier polysaccharide fortement réticulé,

4') éventuellement séchage des particules obtenues lors de l'étape 3').

c) broyage dudit premier polysaccharide réticulé obtenu lors de l'étape a) ou b) en particules de granulométrie souhaitée pour obtenir des particules de premier polysaccharide fortement réticulé;

d) mise en suspension des particules obtenues lors de l'étape c) dans un milieu aqueux pour obtenir une suspension;

e) ajout d'au moins un second polysaccharide, ledit second polysaccharide étant (identique ou différent de celui-ci employé à l'étape a)) à la suspension obtenue lors de l'étape d) ;

f) réticulation dudit au moins second polysaccharide jusqu'à un taux de réticulation X2 pour obtenir au moins un second polysaccharide faiblement réticulé et co-réticulation dudit au moins second polysaccharide faiblement réticulé avec lesdites particules en présence d'au moins un agent réticulant pour obtenir un gel co-réticulé ; et

g) récupération du gel co-réticulé obtenu lors de l'étape f) comprenant des particules d'au moins un premier polysaccharide fortement réticulé, liées de manière covalente à une matrice d'au moins un second polysaccharide faiblement réticulé.

Dans les procédés de préparation ci-dessus, lors de l'étape 4) ou f), les deux étapes de réticulation du au moins second polysaccharide et co-réticulation du au moins second polysaccharide faiblement réticulé avec les particules sont simultanées. Le milieu réactionnel complet obtenu à la fin de l'étape 3) ou e) est soumis à la réticulation, ce milieu comprenant les particules de premier polysaccharide réticulé et au moins un second polysaccharide.

Dans le procédé de préparation du mode de réalisation particulier, les deux étapes de réticulation/co-réticulation a) et f) sont réalisées dans un milieu dont la valeur de pH est sensiblement la même, voire identique. Chacune de ces étapes peut être effectuée en milieu acide ou basique, de préférence en milieu basique, par exemple à un pH compris entre 8 et 14, de préférence entre 8 et 13.

Les réactions de réticulation et co-réticulation mises en oeuvre dans les procédés de l'invention sont des réactions bien connues de l'homme du métier. Pour chaque polysaccharide et/ou agent réticulant, l'homme du métier pourra mettre au point et optimiser les conditions de réticulations en fonction dudit ou desdits polysaccharide(s) et dudit ou desdits agent(s) réticulant(s) : taux de réticulation, température, pH. Il est cependant précisé que les étapes de réticulation et de co-réticulation sont réalisées à pH constant, soit acide, soit basique, comme indiqué précédemment.

Les agents réticulants qui interviennent dans les étapes de réticulation et de co-réticulation 4) et 1') ou a) et f) sont généralement des réticulants bi- ou poly-fonctionnels de différents types, et peuvent par exemple être choisis parmi la DVS (divinylsulfone) en milieu alcalin (voir US 4 582 865), les époxy bi- ou poly-fonctionnels (voir US 4 716 154), les carbodi-imides, le formaldéhyde (voir GB 2 151 244). Les agents réticulants utilisés dans les étapes 4) et 1'), ou a) et f) peuvent être identiques ou différents. De plus, il est possible d'utiliser plus d'un agent réticulant dans les étapes 4), 1'), a) ou f).

On préfère en particulier les agents de type bi- ou poly-époxydes, les réactions se faisant en milieu basique pour générer des liaisons éther avec les fonctions -OH du polysaccharide, ou en milieu acide ce qui donne lieu à des liaisons de type ester. La demande de brevet WO 2000/46253 utilise successivement ces deux conditions de pH afin d'optimiser la réticulation du polysaccharide. On préfère toutefois réaliser les réactions de réticulation et de co-réticulation en conditions de pH basique, puisque, en milieu aqueux, les liaisons ester, obtenues en milieu acide, sont généralement plus labiles que les liaisons éther, obtenues en milieu basique.

À titre d'agent réticulant, on peut utiliser un époxyde ou ses dérivés, et notamment le 1,4-butanedioldiglycidyléther (BDDE), le diépoxy-octane ou le 1,2-bis-(2,3-époxypropyl)-2,3-éthylène. On préfère tout particulièrement utiliser le 1,4-butanedioldiglycidyléther (BDDE) pour chacune des étapes de réticulation et de co-réticulation.

Il doit être compris que chacune des étapes de réticulation et de co-réticulation peut être effectuée avec un ou plusieurs agents réticulants, ceux-ci pouvant être identiques ou différents dans l'une ou l'autre des étapes de (co-)réticulation, dans les conditions de pH indiquées plus haut.

De manière générale, les taux de réticulation X1 et X2 sont exprimés comme indiqués ci-dessus, la caractéristique étant que X1 est très supérieur à X2, comme décrit plus haut.

Après chacune des étapes 4), 1'), a) et/ou f) de réticulation, les polysaccharides fortement réticulé et faiblement réticulé/co-réticulé peuvent avantageusement être purifiés, selon des techniques classiques de purification (par exemple par lavage par flux d'eau continu, bains de dialyse, et autres), afin d'éliminer l'agent de réticulation résiduel n'ayant pas réagi.

En outre, les étapes de réticulation/co-réticulation 4), 1'), a) et f) peuvent avantageusement être suivies d'une étape de neutralisation (i.e. jusqu'à une valeur de pH d'environ 7), par exemple par ajout d'une quantité appropriée d'acide chlorhydrique 1N.

Le broyage du premier polysaccharide de l'étape 3') ou c) peut être effectué selon tout procédé connu dans le domaine, et par exemple peut être un broyage mécanique, notamment au moyen d'un broyeur de type Ultraturrax, ou par cisaillement au cours de la pression à travers des tubes de diamètres intérieurs maîtrisés, pour obtenir les tailles souhaitées selon l'application envisagée.

Selon une première variante du procédé de l'invention, les particules d'un premier polysaccharide fortement réticulé de l'étape 1) sont co-réticulées au sein du gel final sous leur forme hydratée. Elles sont transparentes, ce qui permet l'obtention d'un gel transparent de particules de gel co-réticulées dans le gel final.

Selon une autre variante, le procédé de l'invention met en oeuvre des particules sèches (ou déshydratées) de premier polysaccharide fortement réticulé. Si les particules de premier polysaccharide fortement réticulé sont fournies sous forme hydratées lors de l'étape 1), une étape de séchage (déshydratation) des particules de premier polysaccharide fortement réticulé est ajoutée avant l'étape de mise en suspension des particules pour obtenir des particules sèches. Pour réaliser cette variante, il est également possible de fournir des particules sèches de premier polysaccharide fortement réticulé lors de l'étape 1). Pour obtenir des particules sèches de premier polysaccharide fortement réticulé, une étape de séchage des particules de premier polysaccharide réticulé obtenues lors de l'étape 3') peut être ajoutée au procédé de préparation des particules de premier polysaccharide après l'étape 3') (étape 4'). Une autre alternative est d'effectuer l'étape de séchage avant l'étape de broyage du premier polysaccharide réticulé (étape 2').

Cette étape de séchage peut être effectuée par tous moyens connus de l'homme du métier, par exemple séchage en étuve sous vide, ou à l'air libre sous flux laminaire. Le séchage est avantageusement conduit, mais non nécessairement, jusqu'à déshydratation totale du gel (élimination de l'eau libre).

Dans ce mode de réalisation, lors de l'étape de mise en suspension des particules sèches dans un milieu aqueux, la réhydratation des particules sèches n'est pas complète, ce qui se manifeste par le fait que les particules ne sont pas transparentes.

Lorsque des particules déshydratées sont mises en oeuvre dans le gel selon l'invention, il est apparu que, si la réticulation réalisée génère des liaisons stables (telles que des liaisons éther notamment, voire *supra*), les particules déshydratées ne se réhydratent pas totalement dans le gel final, voire l'hydrogel final, et ce même après un séjour de 2h30 dans l'hydroxyde de sodium à 1 % (poids/poids).

On obtient alors un gel final co-réticulé avec des particules en partie hydratées seulement, des « voiles » blancs, « filaments » blancs sont visibles dans le produit fini. Dans cette variante, le gel selon l'invention comportant des particules de gel fortement réticulé déshydratées ou partiellement déshydratées (ou hydratées) est susceptible de générer un plus grand effet volumateur, par un effet de relance fibroblastique plus important, qu'avec un gel comportant des particules de gel fortement réticulé n'ayant subi aucun séchage avant co-réticulation avec la matrice.

À l'issue de la première réticulation (étape 1') et/ou de la deuxième réticulation/co-réticulation (étape 4), il peut être avantageux de neutraliser le gel obtenu, selon les procédés standards connus dans le domaine, et par exemple par ajout d'acide lorsque la réticulation est conduite en milieu acide, et par ajout d'une base, lorsque la réticulation est conduite en milieu acide.

Dans le procédé selon l'invention, l'étape 2) de mise en suspension des particules dans un milieu aqueux correspond à un mélange des particules sèches ou hydratées dans un milieu aqueux.

Le gel cohésif co-réticulé obtenu selon le procédé décrit ci-dessus est enfin avantageusement soumis à une étape d'hydratation complémentaire, afin d'obtenir un gel co-réticulé sous forme d'hydrogel injectable, adapté aux applications envisagées.

Cette hydratation est généralement effectuée, comme pour l'étape 2) ou d) du procédé décrit plus haut, dans un milieu aqueux, par simple mélange du gel réticulé avec une solution aqueuse, avantageusement physiologique tamponnée, de manière à obtenir une concentration finale, variable dans de très larges proportions, selon la nature des polysaccharides utilisés, leurs taux de réticulation respectifs, et également selon l'utilisation envisagée. La solution tamponnée utilisable peut par exemple être une solution physiologique, iso-osmolaire présentant un pH compris entre environ 6,8 et environ 7,5.

Cette concentration finale en polysaccharides totaux est généralement comprise entre environ 5 et environ 100 mg/g, de préférence entre environ 5 et environ 50 mg/g par exemple environ 20 mg/g d'hydrogel (produit fini). Par «polysaccharides totaux », on désigne l'ensemble formé par le premier et le second polysaccharide.

Le procédé de la présente invention permet ainsi d'obtenir un gel (ou un hydrogel) co-réticulé biocompatible, cohésif, injectable et de rémanence longue. Les particules fortement réticulées (totalement hydratées, ou en partie) au sein de ce gel permettront éventuellement de générer une néo-collagénèse au sein du derme suite à la dégradation de la matrice plus légèrement réticulé.

Ainsi une double action de ce gel peut être escomptée, notamment pour une utilisation esthétique de comblement des rides :

- un comblement immédiat des volumes grâce à l'injection d'un hydrogel monophasique épais au sein de la dépression dermique qu'est la ride,
- une relance du phénomène de synthèse endogène de collagène et une prolifération des fibroblastes suite à la réaction à corps étranger induite par les particules souples de gel fortement réticulé.

De préférence, le premier et/ou le second polysaccharide mis en oeuvre dans le procédé selon l'invention est l'acide hyaluronique ou un de ses sels.

La présente invention propose donc un gel comprenant des particules de polysaccharide(s), préalablement fortement réticulé(s), puis réduites en particules de très fines dimensions, comme indiqué précédemment, au sein d'une matrice de polysaccharide(s) faiblement réticulé(s). Ainsi le gel (ou l'hydrogel, obtenu après hydratation du gel comme décrit plus haut) s'injecte facilement, même à travers des aiguilles de diamètre fin (type 27 Gauge), est cohésif et se répartit facilement dans les tissus, sans former de « paquets », qui sinon seraient difficiles à homogénéiser sous la peau.

En outre, sa cinétique de dégradation est ralentie du fait des particules fortement réticulées qui restent dans le derme beaucoup plus longtemps que les gels réticulés classiques, sans pour autant générer de fortes réactions à particules étrangères comme dans le cas de particules solides et de formes souvent mal maîtrisées. Ainsi, il pourra être observé une prolifération des fibroblastes susceptibles de participer à la création de volumes dans un second temps, augmentant encore la durée de comblement du produit.

Ainsi, et selon un troisième aspect, la présente invention a pour objet les utilisations des gels et hydrogels précédemment définis pour séparer, combler les tissus, augmenter le volume desdits tissus, remplacer un fluide biologique, ou encore pour servir de matrice d'injection à relargage contrôlé à un ou des principe(s) actif(s) dispersé(s).

Les applications visées sont plus particulièrement les applications communément observées dans le cadre des viscoélastiques injectables de polysaccharides utilisés ou potentiellement utilisables dans les pathologies ou traitements suivants :

- injections esthétiques : de comblement des rides, défauts cutanés ou volumatrices (pommettes, mentons, lèvres) ;
- traitement de l'arthrose, injection dans l'articulation en remplacement ou complément du liquide synovial déficient ;
- injection péri-urétrale pour le traitement de l'incontinence urinaire par insuffisance sphinctérienne ;
- injection post chirurgicale pour éviter les adhésions péritonéales notamment ;
- injection suite à une chirurgie de la presbytie par incisions sclérales au laser.

Plus particulièrement, en chirurgie esthétique, et selon la taille et la proportion de particules au sain du gel final co-réticulé, ce dernier pourra être utile :

- pour le comblement des rides fines, moyennes ou profondes, et être injecté avec des aiguilles de diamètre fin (27 Gauge par exemple) ;
- comme volumateur avec une injection par des aiguilles de diamètre plus important, de 22 à 26 Gauge ; dans ce cas, son caractère cohésif permettra de garantir son maintien à l'emplacement de l'injection.

Pour des injections dans l'articulation, des aiguilles de diamètre plus importants sont préférées, par exemple des aiguilles de 18 à 22 Gauge. Par exemple, 1 à 5 mL d'hydrogel peuvent être injectés, de préférence environ 2 mL.

En chirurgie urologique, la taille des particules et leur proportion seront adaptées à l'application et permettront de garantir une rémanence accrue. La cohésivité du produit permettra un comblement efficace de l'organe.

Le gel co-réticulé selon l'invention trouve également une application importante en chirurgie articulaire et en chirurgie dentaire pour le comblement des poches parodontales par exemple.

Ces exemples d'utilisation ne sont nullement limitants, le gel co-réticulé selon la présente invention étant plus largement prévu pour :

- combler des volumes ;
- générer des espaces au sein de certains tissus, favorisant ainsi leur fonctionnement optimal ;
- remplacer des liquides physiologiques déficients.

Le gel co-réticulé selon l'invention peut également trouver une application tout à fait intéressante en tant que matrice de relargage d'un (ou plusieurs) principe(s) actif(s) au préalable dispersé(s) en son sein. Par principe actif on entend tout produit actif sur le plan pharmacologique : principe actif médicamenteux, anti-oxydant, antiseptique, etc...

De manière pratique, le gel selon l'invention, de préférence après purification et hydratation en hydrogel, comme décrit précédemment, peut être conditionné, par exemple dans des seringues, et stérilisé selon tout moyen connu en soi (par exemple par autoclavage) pour commercialisation et/ou utilisation directe.

Selon un autre aspect, la présente invention concerne une trousse (ou kit) pour l'injection d'un gel comprenant un gel co-réticulé selon l'invention, avantageusement sous forme d'hydrogel prêt à l'emploi, conditionné en seringue stérile, ou avec une solution tampon pour préparation extemporanée, ainsi qu'un nécessaire à injection comprenant au moins une aiguille d'injection.

Des exemples de réalisation de la présente invention sont présentés ci-après, et ont pour but d'illustrer l'invention, sans toutefois y apporter une quelconque limitation.

**Exemple 1 :**

Étape a) : *Hydratation de fibres de hyaluronate de sodium sous forme de gel non réticulé*

Des fibres de hyaluronate de sodium de qualité injectable (1 g ; masse moléculaire : environ 1,5 MDa ; sont pesées et préalablement séchées dans un récipient. Une solution aqueuse d'hydroxyde de sodium à 1 % (poids/poids) dans l'eau (6,2 g) est ajoutée, le tout est homogénéisé pendant environ 1 heure à température ambiante.

Étape b) : *Première réticulation*

Du BDDE (150 mg) est ajouté au gel de hyaluronate de sodium (NaHA) non réticulé obtenu à l'étape précédente, le tout étant homogénéisé mécaniquement pendant environ 30 minutes à température ambiante. L'ensemble est ensuite placé au bain-marie à 50°C +/- 2°C pendant 2h15 à 3h, afin d'obtenir un taux de réticulation X1 d'environ 0,32. Le gel de NaHA réticulé est alors placé dans 3 bains successifs d'EDI (eau déionisée) de 8 à 12 h chacun, afin de gonfler et d'éliminer le réticulant résiduel n'ayant pas réagi.

Étape c) : *Broyage du polysaccharide fortement réticulé*

Le gel réticulé hydraté est ensuite broyé à l'Ultra-turrax afin d'obtenir des particules de gel de taille variant entre 0,1 et 3 mm. Les particules de gel sont laissées à égoutter pendant 2 heures sur un tamis de porosité 100 $\mu$m.

Étape d) : *Réticulation du second polysaccharide et co-réticulation*

Les particules hydratées sont pesées (5 g) dans un récipient dans lequel on ajoute 0,8 g de fibres de NaAH sèches (masse moléculaire : environ 1,5 MDa) et 2 g de solution aqueuse d'hydroxyde de sodium à 1,5 % (poids/poids), l'ensemble étant homogénéisé pendant environ 30 minutes à température ambiante.

Au mélange sont alors ajoutés 30 mg de BDDE, puis l'ensemble est homogénéisé mécaniquement pendant 30 minutes environ, avant d'être placé au bain-marie à 50°C +/-2°C pendant 2h à 2h30, afin d'obtenir un taux de réticulation X2 d'environ 0,08.

Le gel final co-réticulé est ensuite neutralisé par ajout d'HCl 1N, et placé dans un bain de tampon phosphate pour stabiliser le pH et permettre son hydratation, ou gonflement. On obtient ainsi un hydrogel co-réticulé conformément à l'invention.

En final, l'hydrogel est placé en poches de dialyse dans divers bains de tampon phosphate afin d'éliminer l'agent réticulant excessif, puis est homogénéisé avant d'être rempli en seringues qui sont stérilisées par autoclavage.

L'hydrogel ainsi obtenu est transparent, injectable à travers des aiguilles hypodermiques 22 Gauge, pour la création de volumes dans les pommettes ou le menton. Sa concentration finale en NaHA est ajustée à 20 mg/g de gel.

**Exemple 2 :**

Les étapes a) et b) de l'exemple 1 sont reproduites dans ce deuxième exemple.

<u>Étape c)</u> : *Broyage et séchage du polysaccharide fortement réticulé*

Le gel réticulé hydraté est ensuite broyé à l'Ultra-turrax afin d'obtenir des particules de gel de taille variant entre 0,1 et 3 mm. Les particules de gel sont laissées à égoutter pendant 2 heures sur un tamis de porosité 100 μm, puis à sécher une nuit en étuve ventilée à 35°C.

<u>Étape d)</u> : *Réticulation du second polysaccharide et co-réticulation*

Les particules déshydratées sont pesées (600 mg) dans un récipient dans lequel on ajoute 5 g de fibres de NaAH sèches (masse moléculaire : environ 1,5 MDa) et 36 g de solution aqueuse d'hydroxyde de sodium à 1 % (poids/poids), l'ensemble étant homogénéisé pendant environ 1 heure à température ambiante.

Au mélange sont alors ajoutés 300 mg de BDDE, puis l'ensemble est homogénéisé mécaniquement pendant 30 minutes environ, avant d'être placé au bain-marie à 50°C +/-2°C pendant 2h à 2h30, afin d'obtenir un taux de réticulation X2 d'environ 0,12.

Le gel final co-réticulé est ensuite neutralisé par ajout d'HCl 1N, et placé dans un bain de tampon phosphate pour stabiliser le pH et permettre son hydratation, ou gonflement. On obtient ainsi un hydrogel co-réticulé conformément à l'invention.

Comme précédemment, l'hydrogel est placé en poches de dialyse dans divers bains de tampon phosphate afin d'éliminer l'agent réticulant excessif, puis est homogénéisé avant d'être rempli en seringues qui sont stérilisées par autoclavage.

L'hydrogel ainsi obtenu est transparent dans l'ensemble, avec des zones de « voiles » blancs, des filaments blanchâtres visibles en son sein, injectable à travers des aiguilles hypodermiques 22 à 27 Gauge pour la création de volumes dans les pommettes ou le menton, ou le comblement des rides profondes. Sa concentration finale en NaHA est de 20 mg/g de gel.

**Exemple 3 : (comparatif)**

L'étape a) de l'exemple 1 est réalisée.

<u>Étape b)</u> :

Du BDDE (70 mg) est ajouté au gel de hyaluronate de sodium (NaHA) non réticulé obtenu à l'étape précédente, le tout étant homogénéisé mécaniquement pendant environ 30 minutes à température ambiante. L'ensemble est ensuite placé au bain-marie à 50°C +/- 2°C pendant 2h15 à 3h, afin d'obtenir un taux de réticulation X1 d'environ 0,15.

Le gel final réticulé est ensuite neutralisé par ajout d'HCl 1N, et placé dans un bain de tampon phosphate pour stabiliser le pH et permettre son hydratation, ou gonflement. On obtient ainsi un hydrogel de NaHa réticulé par la voie classiquement utilisée.

L'hydrogel est ensuite placé en poches de dialyse dans divers bains de tampon phosphate afin d'éliminer l'agent réticulant excessif, et est homogénéisé avant d'être rempli en seringues stérilisées par autoclavage.

La durée d'homogénéisation finale est fixée équivalente à celle du gel réalisé lors de l'exemple 2, afin de se placer dans des situations comparables.

Ce gel final est transparent, sa concentration finale en NaHA est de 20 mg/g de gel.

La comparaison des gels obtenus dans l'exemple 2 (selon l'invention, gel co-réticulé) et dans l'exemple 3 (gel réticulé simple) met en évidence une différence d'aspect : ainsi dans le cas de l'exemple 2, des zones voilées blanchâtres, des filaments blancs sont visibles au sein du gel transparent dans l'ensemble, alors que le gel de l'exemple 3 est totalement transparent.

Les deux hydrogels ont un aspect cohésif lorsqu'ils sont extrudés de la seringue. Par ailleurs, l'hydrogel de l'exemple 2 est plus facile à expulser au travers d'une aiguille 22 Gauge 1/2 que l'hydrogel de l'exemple 3, les deux hydrogels étant conditionnés en seringue 2 mL identiques. Le rapport des forces d'injection obtenues est le suivant :

$$\text{d'injection obtenues est le suivant :}$$

$$F \text{ gel exemple } 3 \, / \, F \text{ gel exemple } 2 = 1,25$$

Ceci confirme une plus grande facilité d'injection de l'hydrogel de l'exemple 2.

Ainsi, pour deux gels de concentration finale 20 mg/g de hyaluronate de sodium, réticulés par une concentration équivalente de BDDE (70 mg BDDE pour 1 g d'acide hyaluronique) mais dans des conditions différentes, on observe une facilité d'injection accrue de l'hydrogel de l'invention, ceci pour une durée d'homogénéisation finale de l'hydrogel équivalente.

Par ailleurs les particules fortement réticulées du gel de l'exemple 2, sont aptes à induire *in fine* une rémanence accrue dans le temps du gel après implantation, avec un phénomène de relance fibroblastique et de néo-collagénase.

Ces deux exemples comparés mettent en évidence l'intérêt de l'hydrogel co-réticulé selon la présente invention.

**Exemple 4 : Gel de hyaluronate de sodium / chondroitine sulfate réticulé sous forme de particules, ensuite co-réticulées au sein d'un gel de NaHa.**

<u>Étape a)</u> : *Hydratation de fibres de hyaluronate de sodium et de chondroitine sulfate de sodium sous forme de gel non réticulé*

Des fibres de hyaluronate de sodium de qualité injectable (0,6 g ; masse moléculaire : environ 1,5 MDa) et de la poudre de chondroitine sulfate de qualité injectable (0,4 g ; masse moléculaire : environ 40 kDa) sont pesées et préalablement séchées dans un récipient. Une solution aqueuse d'hydroxyde de sodium à 1 % (poids/poids) dans l'eau (6,2 g) est ajoutée, le tout est homogénéisé pendant environ 1 heure à température ambiante.

<u>Étape b)</u> : *Première réticulation*

Du BDDE (150 mg) est ajouté au gel de hyaluronate de sodium / chhondroitine sulfate de sodium (NaHa / NaCS) non réticulé obtenu à l'étape précédente, le tout étant homogénéisé mécaniquement pendant environ 30 minutes à température ambiante. L'ensemble est ensuite placé au bain-marie à 50°C +/- 2°C pendant 2h15 à 2h30, afin d'obtenir un taux de réticulation X1 d'environ 0,32. Le gel de NaHA/NaCS réticulé est alors placé dans 3 bains successifs d'EDI (eau désionisée) de 8 à 12 h chacun, afin de gonfler et d'éliminer le réticulant résiduel n'ayant pas réagi.

<u>Étape c)</u> : *Broyage et séchage du gel de polysaccharides fortement réticulés*

Le gel réticulé hydraté est ensuite broyé à l'Ultra-turrax afin d'obtenir des particules de gel de taille variant entre 0,5 et 5 mm. Les particules de gel sont laissées à égoutter pendant 2 heures sur un tamis de porosité 100 $\mu$m, puis à sécher une nuit en étuve ventilé à 35°C.

<u>Étape d)</u> : *Réticulation du second polysaccharide et co-réticulation*

Les particules déshydratées sont pesées (800 mg) dans un récipient dans lequel on ajoute 4,2 g de fibres de NaAH sèches (masse moléculaire : environ 1,5 MDa) et 36 g de solution aqueuse d'hydroxyde de sodium à 1 % (poids/poids), l'ensemble étant homogénéisé pendant environ 1 heure à température ambiante.

Au mélange sont alors ajoutés 300 mg de BDDE, puis l'ensemble est homogénéisé mécaniquement pendant 30 minutes environ, avant d'être placé au bain-marie à 50°C +/-2°C pendant 2h à 2h30, afin d'obtenir un taux de réticulation X2 d'environ 0,12.

Le gel final co-réticulé est ensuite neutralisé par ajout d'HCl 1N, et placé dans un bain de tampon phosphate pour stabiliser le pH et permettre son hydratation, ou gonflement. On obtient ainsi un hydrogel co-réticulé conformément à l'invention.

La suite des étapes est équivalente à celle des exemples précédents.

La concentration finale de l'hydrogel est ajustée à 25 mg/g en NaHA / NaCS.

L'hydrogel ainsi obtenu est transparent avec des fragments blanchâtres répartis de manière homogène en son sein. Il est injectable à travers des aiguilles hypodermiques 22 Gauge, pour la création de volumes dans les fesses, les pommettes, le menton, etc...ou encore pour l'injection dans des poches parodontales.

**Exemple 5 : gel de HPMC réticulé sous forme de particules ensuite co-réticulées au sein d'un gel de NaHa.**

Étape a) : *Hydratation d'HPMC sous forme de gel non réticulé*

De la poudre d'Hydroxypropyl méthylcellulose (HPMC) de qualité injectable (1 g) est pesée et préalablement séchée dans un récipient. Une solution aqueuse d'hydroxyde de sodium à 1 % (poids/poids) dans l'eau (5.5 g) est ajoutée, le tout est homogénéisé pendant environ 1 heure et 30 minutes à température ambiante.

Étape b) : *Réticulation de l'HPMC*

Du BDDE (165 mg) est ajouté au gel d'hydroxypropylméthylcellulose (HPMC) non réticulé obtenu à l'étape précédente, le tout étant homogénéisé mécaniquement pendant environ 30 minutes à température ambiante. L'ensemble est ensuite placé au bain-marie à 45°C +/- 2°C pendant 3h à 3h30. Le gel d'HPMC réticulé est alors placé dans 3 bains successifs d'EDI (eau désionisée) de 8 à 12 h chacun, afin de gonfler et d'éliminer le réticulant résiduel n'ayant pas réagi.

Étape c) : *Broyage du gel d'HPMC fortement réticulé*

Le gel réticulé hydraté est ensuite broyé à l'Ultra-turrax afin d'obtenir des particules de gel de taille variant entre 0,2 et 2 mm. Les particules de gel sont laissées à égoutter pendant 2 heures sur un tamis de porosité 100 $\mu$m. Étape d) : *Réticulation du second polysaccharide et co-réticulation*

Les particules hydratées sont pesées (5 g) dans un récipient dans lequel on ajoute 0,8 g de fibres de NaAH sèches (masse moléculaire : environ 1,5 MDa) et 2 g de solution aqueuse d'hydroxyde de sodium à 1,5 % (poids/poids), l'ensemble étant homogénéisé pendant environ 30 minutes à température ambiante.

Au mélange sont alors ajoutés 30 mg de BDDE, puis l'ensemble est homogénéisé mécaniquement pendant 30 minutes environ, avant d'être placé au bain-marie à 50°C +/-2°C pendant 2h à 2h30, afin d'obtenir un taux de réticulation X2 d'environ 0,08.

Le gel final co-réticulé est ensuite neutralisé par ajout d'HCl 1 N, et placé dans un bain de tampon phosphate pour stabiliser le pH et permettre son hydratation, ou gonflement. On obtient ainsi un hydrogel co-réticulé conformément à l'invention.

Finalement, l'hydrogel est placé en poches de dialyse dans divers bains de tampon phosphate afin d'éliminer l'agent réticulant excessif, puis est homogénéisé avant d'être rempli en seringues qui sont stérilisées par autoclavage.

L'hydrogel ainsi obtenu est transparent, injectable à travers des aiguilles hypodermiques 22 Gauge, pour la création de volumes en injection sous-cutanée. Sa concentration finale en polysaccharides est ajustée à 25 mg/g de gel.

**Revendications**

1. Gel cohésif co-réticulé injectable, comprenant au moins un premier gel de polysaccharide fortement réticulé possédant un taux de réticulation X1' compris entre 0,21 et 0,9 sous forme de particules réparties de manière homogène dans au moins un second gel de polysaccharide faiblement réticulé, possédant un taux de réticulation X2 compris entre 0,03 et 0,3, ledit au moins premier gel étant lié par liaisons de covalence audit au moins second gel, le taux de réticulation dudit polysaccharide fortement réticulé étant supérieur au taux de réticulation dudit polysaccharide faiblement réticulé dans lequel :

- ledit polysaccharide fortement réticulé et ledit polysaccharide faiblement réticulé sont choisis parmi l'acide hyaluronique ou un de ses sels, la chondroïtine sulfate ou un de ses sels et l'hydroxypropylmethylcellulose et l'un au moins desdits polysaccharides est l'acide hyaluronique ou un de ses sels,
- les taux de réticulation X1' et X2 sont définis comme étant égal au rapport :

$$X = \frac{nombre\ de\ moles\ de\ réticulant\ introduites\ dans\ le\ milieu\ réactionnel}{nombre\ total\ de\ motif\ dissaccharide\ introduits\ dans\ le\ milieu\ récationnel}$$

- le premier gel fortement de réticulé est sous forme de particules réparties de manière homogène dans le second gel ;
- lesdites particules de polysaccharide fortement réticulé sont liées de manière covalente au polysaccharide faiblement réticulé ;
- la proportion pondérale desdites particules de polysaccharide fortement réticulé possédant un taux de réticulation X1' compris entre 0,21 et 0,9 est comprise entre 0,5 à 99 % ;
- la taille desdites particules est comprise entre 10 $\mu$m et 5 mm.

2. Gel selon la revendication 1, dans lequel le polysaccharide du second gel est identique au polysaccharide du premier gel de polysaccharide.

3. Gel selon la revendication 1, dans lequel le polysaccharide du second gel et le polysaccharide du premier gel de polysaccharide sont différents.

4. Composition comprenant un gel selon l'une quelconque des revendications 1 à 3.

5. Procédé de préparation d'un gel selon l'une quelconque des revendications précédentes, **caractérisé en ce qu'**il comprend les étapes de :

    1) fournir des particules d'un premier polysaccharide choisi parmi l'acide hyaluronique ou un de ses sels, la chondroïtine sulfate ou un de ses sels et l'hydroxypropylméthylcellulose possédant un taux de réticulation X1 compris entre 0,2 et 0,7 ;
    2) mise en suspension des particules de l'étape 1) dans un milieu aqueux pour obtenir une suspension;
    3) ajout d'au moins un second polysaccharide choisi parmi l'acide hyaluronique ou un de ses sels, la chondroïtine sulfate ou un de ses sels et l'hydroxypropylméthylcellulose, ledit second polysaccharide étant identique ou différent du premier polysaccharide, à la suspension obtenue lors de l'étape 2) ;
    4) réticulation dudit au moins second polysaccharide jusqu'à un taux de réticulation X2 pour obtenir au moins un second polysaccharide possédant un taux de réticulation compris entre 0,03 et 0,3 et co-réticulation dudit au moins second polysaccharide possédant un taux de réticulation compris entre 0,03 et 0,3 avec lesdites particules en présence d'au moins un agent réticulant pour obtenir un gel co-réticulé dans lequel au moins un desdits polysaccharides est l'acide hyaluronique ou un de ses sels ; et
    5) récupération du gel co-réticulé obtenu lors de l'étape 4) comprenant des particules d'au moins un premier polysaccharide possédant un taux de réticulation X1' compris entre 0,21 et 0,9, liées de manière covalente à une matrice d'au moins un second polysaccharide possédant un taux de réticulation compris entre 0,03 et 0,3.

6. Procédé de préparation d'un gel selon la revendication 5, dans lequel les particules de premier polysaccharide possédant un taux de réticulation X1 compris entre 0,2 et 0,7 sont sèches lors de la mise en suspension de l'étape 2).

7. Procédé selon l'une quelconque des revendications 5 à 6, **caractérisé en ce que** les agents réticulants sont des agents réticulants bi- ou poly-fonctionnels.

8. Procédé selon l'une quelconque des revendications 5 à 7, **caractérisé en ce que** le gel co-réticulé obtenu est soumis à une étape d'hydratation complémentaire, afin d'obtenir un produit fini de concentration en polysaccharide totaux comprise entre 5 et 100 mg/g.

9. Utilisation d'un gel co-réticulé selon l'une quelconque des revendications 1 à 3, pour séparer, combler les tissus, augmenter le volume desdits tissus, remplacer un fluide biologique, ou encore pour servir de matrice d'injection à relargage contrôlé à un ou des principe(s) actif(s) dispersé(s).

10. Utilisation d'un gel co-réticulé selon la revendication 9 pour le comblement des rides fines, moyennes ou profondes et des défauts cutanés.

11. Trousse pour l'injection d'un gel comprenant un gel co-réticulé selon l'une quelconque des revendications 1 à 3, conditionné en seringue stérile, ainsi qu'un nécessaire à injection comprenant au moins une aiguille d'injection.

**Patentansprüche**

1. Haftfähiges co-vernetztes injizierbares Gel umfassend wenigstens ein erstes, stark vernetztes Polysaccharidgel mit einem Vernetzungsgrad X1' von 0,21 bis 0,9 in Form von Partikeln, die homogen verteilt sind in wenigstens einem zweiten, schwach vernetzten Polysaccharidgel mit einem Vernetzungsgrad X2 von 0,03 bis 0,3, wobei das wenigstens erste Gel kovalent mit dem wenigstens zweiten Gel verbunden ist, der Vernetzungsgrad des stark vernetzten Polysaccharids größer ist als der Vernetzungsgrad des schwach vernetzten Polysaccharids, wobei
   das stark vernetzte Polysaccharid und das schwach vernetzte Polysaccharid ausgewählt sind aus Hyaluronsäure oder einem ihrer Salze, Chondroitinsulfat oder einem seiner Salze und Hydroxypropylmethylcellulose, und wenigstens eines der Polysaccharide Hyaluronsäure oder eines ihrer Salze ist;
   die Vernetzungsgrade X1' und X2 gemäß dem folgenden Verhältnis definiert sind:

$$X = \frac{\text{Anzahl der Mole des in das Reaktionsmilieu eingeführten Vernetzungsmittels}}{\text{Gesamtzahl von in das Reaktionsmilieu eingeführten Disaccharideinheiten}}$$

   das erste, stark vernetzte Gel als Partikel vorliegt, die homogen in dem zweiten Gel verteilt vorliegen;
   die Partikel aus stark vernetztem Polysaccharid kovalent mit dem schwach vernetzten Polysaccharid verbunden sind;
   das Gewichtsverhältnis der Partikel aus stark vernetztem Polysaccharid mit einem Vernetzungsgrad X1' von 0,21 bis 0,9 von 0,5 bis 99 % beträgt; und
   die Größe der Partikel von 10 μm bis 5 mm beträgt.

2. Gel nach Anspruch 1, wobei das Polysaccharid des zweiten Gels identisch ist mit dem Polysaccharid des ersten Polysaccharidgels.

3. Gel nach Anspruch 1, wobei das Polysaccharid des zweiten Gels und das Polysaccharid der ersten Polysaccaridgels verschieden sind.

4. Zusammensetzung umfassend ein Gel nach einem der Ansprüche 1 bis 3.

5. Verfahren zum Herstellen eines Gels nach einem der vorangehenden Ansprüche, **dadurch gekennzeichnet, dass** es die folgenden Schritte umfasst:

   1) Bereitstellen von Partikeln aus einem ersten Polysaccharid, das ausgewählt ist aus Hyaluronsäure oder einem ihrer Salze, Chondroitinsulfat oder einem seiner Salze und Hydroxypropylmethylcellulose, mit einem Vernetzungsgrad X1 von 0,2 bis 0,7;
   2) Suspendieren von Partikeln von Schritt 1) in einem wässrigen Milieu, um eine Suspension zu erhalten;
   3) Hinzufügen zu der im Schritt 2) erhaltenen Suspension wenigstens eines zweiten Polysaccharids, das ausgewählt ist aus Hyaluronsäure oder einem ihrer Salze, Chondroitinsulfat oder einem seiner Salze und Hydroxypropylmethylcellulose, wobei das zweite Polysaccharid identisch mit oder verschieden von dem ersten Polysaccharid ist;
   4) Vernetzen des wenigstens zweiten Polysaccharids bis zu einem Vernetzungsgrad X2, um wenigstens ein zweites Polysaccharid mit einem Vernetzungsgrad von 0,03 bis 0,3 zu erhalten, und Co-Vernetzen des wenigstens zweiten Polysaccharids mit einem Vernetzungsgrad von 0,03 bis 0,3 mit den Partikeln in Gegenwart von wenigstens einem Vernetzungsmittel, um ein co-vernetztes Gel zu erhalten, in dem wenigstens eines der Polysaccharide Hyaluronsäure oder eines ihrer Salze ist; und
   5) Wiedergewinnen des in Schritt 4) erhaltenen co-vernetzten Gels umfassend Partikel aus wenigstens einem ersten Polysaccharid mit einem Vernetzungsgrad X1' von 0,21 und 0,9, die kovalent mit einer Matrix aus wenigstens einem zweiten Polysaccharid mit einem Vernetzungsgrad von 0,03 bis 0,3 verbunden sind.

6. Verfahren zum Herstellen eines Gels nach Anspruch 5, wobei die Partikel aus erstem Polysaccharid mit einem Vernetzungsgrad X1 von 0,2 bis 0,7 während des Suspendierens in Schritt 2) trocken sind.

7. Verfahren nach einem der Ansprüche 5 bis 6, **dadurch gekennzeichnet, dass** die Vernetzungsmittel bi- oder polyfunktionelle Vernetzungsmittel sind.

8. Verfahren nach einem der Ansprüche 5 bis 7, **dadurch gekennzeichnet, dass** das erhaltene co-vernetzte Gel einem komplementären Hydratisierungsschritt unterzogen wird, um ein Endprodukt mit einer Gesamtpolysaccharidkonzentration von 5 bis 100 mg/g zu erhalten.

9. Verwendung eines co-vernetzten Gels nach einem der Ansprüche 1 bis 3, zum Trennen, Auffüllen von Geweben, Erhöhen des Volumens der Geweben, Ersetzen einer biologischen Flüssigkeit, oder um eine Injektionsmatrix für kontrollierte Freisetzung eines oder mehrerer dispergierten/dispergierter Wirkstoffe(s) bereitzustellen.

10. Verwendung eines co-vernetzten Gels nach Anspruch 9 zum Auffüllen von zarten, ausgeprägten oder tiefen Falten und Hautdefekten.

11. Set zum Injizieren eines Gels umfassend ein co-vernetztes Gel nach einem der Ansprüche 1 bis 3, verpackt in einer sterilen Spritze, sowie ein Injektionsmittel umfassend wenigstens eine Injektionsnadel.

**Claims**

1. Injectable co-crosslinked cohesive gel comprising at least a first highly crosslinked polysaccharide gel having a crosslinking rate X1' of between 0.21 and 0.9 in the form of homogeneously distributed particles in at least a second slightly crosslinked polysaccharide gel having a crosslinking rate X2 of between 0.03 and 0.3, said at least first gel being bound by covalent bonds to said at least second gel, the crosslinking rate of said highly crosslinked polysaccharide being greater than the crosslinking rate of said slightly crosslinked polysaccharide in which:

   - said highly crosslinked polysaccharide and said slightly crosslinked polysaccharide are selected from hyaluronic acid or a salt thereof, chondroitin sulfate or a salt thereof and hydroxypropylmethylcellulose and at least one of said polysaccharides is a hyaluronic acid or a salt thereof,
   - the crosslinking rates X1' and X2 are defined as the ratio:

$$X = \frac{\text{number of moles of crosslinking agent introduced into the reaction medium}}{\text{total number of disaccharide units introduced into the reaction medium}}$$

   - the first highly crosslinked gel is in the form of homogeneously distributed particles in the second gel;
   - said highly crosslinked polysaccharide particles are covalently bonded to the slightly crosslinked polysaccharide;
   - the weight proportion of said highly crosslinked polysaccharide particles having a crosslinking rate X1' of between 0.21 and 0.9 is between 0.5 to 99%;
   - the size of said particles is between 10 $\mu$m and 5 mm.

2. Gel according to claim 1, wherein the polysaccharide of the second gel is identical to the polysaccharide of the first polysaccharide gel.

3. Gel according to claim 1, wherein the polysaccharide of the second gel and the polysaccharide of the first polysaccharide gel are different.

4. A composition comprising a gel according to any one of claims 1 to 3.

5. Process for preparing a gel according to any one of the preceding claims, **characterized in that** it comprises the steps of:

   1) providing particles of a first polysaccharide selected from hyaluronic acid or a salt thereof, chondroitin sulfate or a salt thereof and hydroxypropylmethylcellulose having a crosslinking rate X1 of between 0.2 and 0.7;
   2) the suspension of the particles in step 1) in an aqueous medium to obtain a suspension;
   3) adding at least a second polysaccharide selected from hyaluronic acid or a salt thereof, chondroitin sulfate or a salt thereof and hydroxypropylmethylcellulose, said second polysaccharide being Identical or different from the first polysaccharide, to the suspension obtained in step 2);
   4) crosslinking at least said second polysaccharide to a crosslinking rate X2 to obtain at least a second polysaccharide having a rate of crosslinking of between 0.03 and 0.3 and co-crosslinking of at least said second polysaccharide having a crosslinking rate of between 0.03 and 0.3 with said particles in the presence of at least one crosslinking agent to obtain a co-crosslinked gel wherein at least one of said polysaccharides is hyaluronic acid or one its salts; and

5) recovering the co-crosslinked gel obtained in step 4) comprising particles of at least a first polysaccharide having a crosslinking rate of X1' of between 0.21 and 0.9, covalently bonded to a matrix of at least a second polysaccharide having a crosslinking rate of between 0.03 to 0.3.

6. Process for preparing a gel according to claim 5, wherein the particles of the first polysaccharide having a crosslinking rate of X1 of between 0.2 and 0.7 are dry during the suspension process in step 2).

7. Process according to any one of claims 5 to 6, **characterized in that** the crosslinking agents are bi- or polyfunctional.

8. Process according to any one of claims 5 to 7, **characterized in that** the co-crosslinked gel obtained is subjected to an additional hydration step in order to obtain a finished product with a total polysaccharide concentration of between 5 and 100 mg/g.

9. Use of a co-crosslinked gel according to any one of claims 1 to 3, to separate, fill the tissues, increase the volume of said tissues, replace a biological fluid, or to serve as an injection matrix of controlled release for one or several dispersed active ingredient(s).

10. Use of a co-crosslinked gel according to claim 9 for filling fine, moderate or deep wrinkles and cutaneous defects.

11. A kit for the injection of a gel comprising a co-crosslinked gel according to any one of claims 1 to 3, packaged in a sterile syringe, and an injection set comprising at least one injection needle.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CA 2561685
- EP 0466300 A
- FR 2865737
- US 4582865 A
- US 4716154 A
- GB 2151244 A
- WO 200046253 A

**Littérature non-brevet citée dans la description**

- **K. DE BOULLE.** Management of complications after implantation of fillers. *Journal of Cosmet. Derm.,* 2004, vol. 3, 2-15
- **C. RUDOLPH.** Foreign body granulomas due to injectable aesthetic microimplants. *P. Soyer, Surg. Pathol.,* 1999, vol. 23, 113-7
- **L. CHRISTENSEN ; V. BREITING.** Adverse reactions to injectable soft tissue permanent fillers. *Aesthetic Plastic Surgery,* 2005, vol. 29, 34-48
- *APS,* 2005, vol. 29, 34-48